# EUROPEAN PATENT SPECIFICATION

(11) **EP 2 611 459 B1**
(45) Date of publication and mention of the grant of the patent: **11.12.2019**
(21) Application number: 11821217.4
(22) Date of filing: 30.08.2011
(51) Int. Cl.: A61K 38/57, A61P 29/00, A61P 11/06, A61P 25/28, A61P 37/06

(54) **Alpha-1 antitrypsin-derived polypeptides for use in the treatment of inflammation**
Alpha-1 Antitrypsin abgeleitete Polypeptide zur Verwendung in der Behandlung von Entzündung
Polypeptides dérivés d'alpha-1 antitrypsin pour l'utilisation dans le traitement de l'inflammation

(30) Priority: 20.12.2010 US 201061425038 P; 31.08.2010 US 378414 P
(43) Date of publication of application: 10.07.2013
(73) Proprietor: Yissum Research Development Company of the Hebrew University of Jerusalem, Ltd., Jerusalem 91390 (IL)
(72) Inventor: WORMSER, Uri, 9720110 Jerusalem (IL)
(74) Representative: Straus, Alexander
(86) International application number: PCT/IL2011/000696
(87) International publication number: WO 2012/029061

(56) References cited:
- EP-A1- 2 105 740
- WO-A2-03/058021
- WO-A2-2009/074350
- WO-A2-2010/054195
- JP-A- 2010 190 804
- US-A1- 2008 095 806
- US-B1- 6 537 968

## Description

### FIELD OF INVENTION

The present invention relates to isolated polypeptides comprising the amino acid sequence of residues 378-413 of *Mus musculus* endogenous α-1-antitrypsin (serpin a1c) and to pharmaceutical compositions comprising same useful for treating inflammatory, autoimmune and degenerative diseases

### BACKGROUND OF THE INVENTION

Systemic Lupus Erythematosus (SLE) is a disease that can produce fever, rash, hair loss, arthritis, pleuritis, pericarditis, nephritis, anemia, leucopenia, thrombocytopenia and central nervous system damage. The clinical course is characterized by periods of remissions and acute or chronic relapses. Characteristic immune abnormalities, especially antibodies to nuclear and other cellular antigens, develop in patients with SLE (Schur PH, systemic lupus erythematosus, in Cecil textbook of Medicine, 22nd edition, Editors: Goldman L, Ausiello D, Saunders, USA, 2004, pp1660-1670).

SLE occurs more frequently in women. The female to male ratio ranges from 10:1 to 15:1 in adults and in other individuals it is 8:1. Among the general population the prevalence is estimated to be 40-50 per 100,000. The cause of SLE remains unknown in spite of many observations on the involvement of genetic, immunologic, hormonal and environmental factors. SLE is primarily a disease with abnormalities of immune system regulation. These abnormalities are secondary to a loss of self tolerance, resulting in autoimmune responses. Due to decreased number of regulatory (suppressor) T cells which control the immune response, the immune response against self components increases, causing activation and proliferation of autoreactive B cells which differentiate into antibody-producing cells and make an excess of antibodies to many nuclear antigens. Anti nuclear antibodies are elevated especially to dsDNA, ssDNA, nucleoproteins and other self components. In addition, female hormones promote B-cell hyperactivity while androgens have the opposite effect. Environmental factors like microorganisms may stimulate cells in the immune system. Ultraviolet irradiation is known to exacerbate lupus skin lesions.

The pathogenesis of SLE includes many manifestations, which are mediated by antibodies. Diffuse proliferative glomerulonephritis is caused by immune complexes, which consist of nuclear antigens and antinuclear antibodies, formed in the circulations and deposited in the glomerular basement membrane or formed in situ. This activates the complement system resulting in the generation of chemotactic factors causing attraction and infiltration of leukocytes which phagocytose the immune complexes and cause the release of mediators which further perpetuate the glomerular inflammation. Deposition of immune complexes leads to chronic inflammation, fibrinoid necrosis and scarring, and renal dysfunction (Schur PH, systemic lupus erythematosus, in Cecil textbook of Medicine, 22nd edition, Editors: Goldman L, Ausiello D, Saunders, USA, 2004, pp1660-1670). Immune complexes have been detected at the dermal-epidermal junction in skin lesions and normal skin, in the choroid plexus, pericardium and pleural cavity.

The pathological symptoms of SLE include renal disease, musculoskeletal manifestations such as arthralgia and arthritis, mucocutaneous lesions including photosensitivity, rash and discoid lesions, vascular, cardiovascular and pulmonary lesions, hematologic manifestations, gastrointestinal and neuropsychiatric disturbances, and general symptoms like sleep disturbances and depression. Drug therapy is mainly based on steroids (prednisone) and immunosuppressants (cyclophosphamide, azathioprine). The prognosis of SLE patients has improved during the last 50 years i.e. the current survival rate is approximately 90% at 10 years. Nevertheless, there are side effects of the medicaments associated with poor quality of life and, in 10% of the cases a bad prognosis is anticipated especially in CNS involvement, hypertension, azotemia and early age of onset.

U.S. Patent No. 6,537,968 discloses a method for treating lupus erythematosus comprising administering a therapeutically effective amount of a composition containing a protease inhibitor selected from a group consisting of alpha 1-antitrypsin, secretory leukocyte protease inhibitor and alpha 2-macroglobulin.

U.S. Patent No. 7,419,670 discloses viral protein SERP-1, SERP-1 analogs or biologically active fragments, which are useful for treating inflammatory or immune reaction associated with arthritis, systemic lupus erythematosus (SLE), multiple sclerosis (MS) and asthma. While U.S. Patent No. 7,419,670 claims methods of treating a mammalian subject having arthritis, systemic lupus erythematosus (SLE), multiple sclerosis (MS) and asthma the polypeptides disclosed are useful only when administered in combination with an immunosuppressant.

U.S. Patent Application No. 2008/0261868 provides a method of treating a subject suffering from a disease characterized by excessive apoptosis by administering at least one serine protease inhibitor, preferably alpha 1-antitrypsin or a derivative thereof.

U.S. Patent Application No. 2008/0095806 discloses protease inhibitor composition useful for preventing and treating hyperproliferative and inflammatory mucocutaneous disorders. U.S. Patent Application No. 2008/0095806 claims methods of treating hyperproliferative and inflammatory mucocutaneous disorders comprising administering to the subject an effective amount of protease inhibitor in a pharmaceutically acceptable carrier or diluent. According to U.S. Patent Application No. 2008/0095806, the protease inhibitor is preferably a serine protease inhibitor, and more preferably alpha 1-antitrypsin including peptide fragments and derivatives thereof useful for preventing and treating hyperproliferative and inflammatory mucocutaneous. Nonetheless there is no indication of specific peptide fragments derived from alpha 1-antitrypsin.

U.S. Patent No. 5,093,316 discloses a method and pharmaceutical compositions for treating pulmonary inflammation in pulmonary diseases comprising administering an effective amount of microcrystalline alpha-1-antitrypsin, derivatives or salts thereof.

International Patent Application No. WO 9206706 provides use of an effective amount of alpha 1-antitrypsin among other serine protease inhibitors for the prophylaxis or treatment of a mast cell-implicated disease or injury in a mammal.

U.S. Patent No. 5,134,119 discloses a method for prophylaxis or direct treatment of mast cell implicated skin inflammation or treating the symptoms of burns in a patient comprising administering an effective amount of an analog of alpha 1-antitrypsin. While U.S. Patent No. 5,134,119 discloses various analogs of alpha 1-antitrypsin, the polypeptides are useful for treatment of mast cell implicated skin inflammation only when methionine at position 358 is substituted with an aliphatic amino acid.

EP 2105740 discloses biomarkers for determining the c-myc activity in a subject, and the use thereof for predicting and monitoring therapeutic intervention in cancer patients. Among others, the 22-mer peptide FDHPFLFIIFEEHTQSPLFVGK is disclosed.

WO 2009/074350 discloses biomarkers for determining the EGFR kinase activity in a subject, and the use thereof for predicting and monitoring therapeutic intervention in cancer patients. Among others, the 22-mer peptide FDHPFLFIIFEEHTQSPLFVGK is disclosed.

WO 2003/058021 discloses nucleic acid molecules coding for (poly)peptides associated with apoptosis. Among others, a 55-mer peptide comprising the sequence YSMPPIVRFDHPFLFIIFEEHTQSPLFVGKVVDPTHK is disclosed.

JP 2010-190804 discloses evaluation of prophylactic and therapeutic effects of test substances against arteriosclerosis, involving administering the test substances to animal model, measuring concentration of marker substances, and comparing measured value with reference value. Among others, the 36-mer peptide SMPPIVRFDHPFLFIIFEEHTQSPLFVGKVVDPTHK and the 22-mer peptide FDHPFLFIIFEEHTQSPIFVGK are disclosed.

There is still an unmet need for improved medicaments for treating inflammatory and autoimmune diseases.

Severe sepsis occurs when the inflammatory reaction towards an infectious agent leads to organ dysfunction, such as trouble breathing, coagulation or other blood abnormalities, decreased urine production, or altered mental status. If the organ dysfunction of severe sepsis is low blood pressure (hypotension), or insufficient blood flow (hypoperfusion) to one or more organs (causing, for example, lactic acidosis), this condition is referred to as septic shock.

Sepsis can lead to multiple organ dysfunction syndrome (MODS) culminating in death. Organ dysfunction results from local changes in blood flow, from sepsis-induced hypotension (< 90 mmHg or a reduction of ≥ 40 mmHg from baseline) and from diffuse intravascular coagulation, among other things.

Sepsis can be defined as the body's response to an infection caused by microorganisms or bacteria invading the body, and can be limited to a particular body region or can be widespread in the bloodstream. Sepsis is acquired quickest with infections that are developed during surgery and physical contact with someone with sepsis.

The therapy of sepsis rests on antibiotics, surgical drainage of infected fluid collections, fluid replacement and appropriate support for organ dysfunction. This may include hemodialysis in kidney failure, mechanical ventilation in pulmonary dysfunction, transfusion of blood products, and drug and fluid therapy for circulatory failure.

Most therapies aimed at the inflammation process itself have failed to improve outcome, however drotrecogin alfa (activated protein C, one of the coagulation factors) has been shown to decrease mortality from about 31% to about 25% in severe sepsis. To qualify for drotrecogin alfa, a patient must have severe sepsis or septic shock with an APACHE II score of 25 or greater and a low risk of bleeding. However, since further trials have failed to replicate this result, the use of activated protein C is controversial.

In some cases, sepsis may lead to inadequate tissue perfusion and necrosis. As this may affect the extremities, amputation may become necessary.

### SUMMARY OF THE INVENTION

The present invention provides isolated polypeptides consisting of an amino acid sequence selected from the group consisting of SEQ ID NO: 1, SEQ ID NO: 2, SEQ ID NO: 3, and SEQ ID NO: 4 and pharmaceutical compositions comprising same for treating diseases attributable to inflammatory processes including autoimmune diseases such as Systemic Lupus Erythematosus (SLE), multiple sclerosis (MS) and arthritis as well as inflammatory pathologies such as sepsis.

It is now disclosed for the first time that a 36-mer peptide designated herein below UBE of the amino acid sequence Ser-Met-Pro-Pro-Ile-Val-Arg-Phe-Asp-His-Pro-Phe-Leu-Phe-Ile-Ile-Phe-Glu-Glu-His-Thr-Gln-Ser-Pro-Leu-Phe-Val-Gly-Lys-Val-Val-Asp-Pro-Thr-His-Lys (SEQ ID NO: 2) corresponding to residues 378-413 of *Mus Musculus* alpha-1-antitrypsin (also designated serpin a1c) is highly effective in eliminating neurological damage in multiple sclerosis as well as in reducing various manifestations of systemic lupus erythematosus (SLE) and arthritis in animal models as well as increasing survival in an animal model of sepsis .It is further disclosed that a peptide designated herein below UBE1 of the amino acid sequence Tyr-Ser-Met-Pro-Pro-Ile-Val-Arg-Phe-Asp-His-Pro-Phe-Leu-Phe-Ile-Ile-Phe-Glu-Glu-His-Thr-Gln-Ser-Pro-Leu-Phe-Val-Gly-Lys-Val-Val-Asp-Pro-Thr-His-Lys (SEQ ID NO: 1) corresponding to residues 377-413 of *Mus Musculus* alpha-1-antitrypsin (serpin a1c) is highly effective in eliminating neurological damage in animal models of multiple sclerosis as well as in reducing joint swelling in arthritic animals.

It is yet further disclosed that a peptide designated herein below UBE-N of the amino acid sequence Met-Pro-Pro-Ile-Val-Arg-Phe-Asp-His-Pro-Phe-Leu-Phe-Ile-Ile-Phe-Glu-Glu-His-Thr-Gln-Ser-Pro-Leu-Phe-Val-Gly-Lys-Val-Val-Asp-Pro-Thr-His-Lys (SEQ ID NO: 3) corresponding to residues 379-413 of *Mus Musculus* alpha-1-antitrypsin (serpin a1c) and a peptide designated herein below UBE-C of the amino acid sequence Ser-Met-Pro-Pro-Ile-Val-Arg-Phe-Asp-His-Pro-Phe-Leu-Phe-Ile-Ile-Phe-Glu-Glu-His-Thr-Gln-Ser-Pro-Leu-Phe-Val-Gly-Lys-Val-Val-Asp-Pro-Thr-His (SEQ ID NO: 4) corresponding to residues 378-412 of *Mus Musculus* alpha-1-antitrypsin (serpin a1c) are highly effective in eliminating neurological damages in animal models of multiple sclerosis.

While the principles of the present invention are exemplified herein below with polypeptides derived from mouse alpha-1 antitrypsin useful in treating inflammatory and autoimmune diseases in animal models or used for treatment of sepsis and asthma in animal models, the present invention encompasses homologous polypeptides derived from human alpha-1 antitrypsin, particularly useful for treating inflammatory and autoimmune diseases in humans.

According to a first aspect, the present disclosure relates to an isolated polypeptide comprising a amino acid sequence sharing at least 95% identity with that set forth in SEQ ID NO:1, or a fragment thereof, or in some embodiments, the amino acid sequence shares at least 97% identity with that set forth in SEQ ID NO: 1, or in some embodiments, the amino acid sequence shares at least 99% identity with that set forth in SEQ ID NO: 1. In some embodiments, the amino acid sequence consists of that set forth in SEQ ID NO: 1.

In some embodiments, the present disclosure relates to a polypeptide comprising an amino acid sequence sharing at least 97% identity with that set forth in SEQ ID NO:2 or a fragment thereof, or in some embodiments, the amino acid sequence shares at least 99% identity with that set forth in SEQ ID NO:2. In some embodiments, the amino acid sequence consists of that set forth in SEQ ID NO:2.

In some embodiments, the present disclosure relates to a polypeptide comprising an amino acid sequence sharing at least 97% identity with that set forth in SEQ ID NO: 3 or 4, or in some embodiments, the amino acid sequence shares at least 99% identity with that set forth in SEQ ID NO: 3 or 4. In some embodiments, the amino acid sequence consists of that set forth in SEQ ID NO: 3 or 4. In some embodiments, the polypeptide is a fragment of the polypeptide of claim 1.

The polypeptides of the present invention corresponding to residues 378-413 of alpha-1 antitrypsin can have at least one amino acid deletion, or, in some embodiments, at least 2, or in some embodiments, at least 3, or in some embodiments, at least 4, or in some embodiments, at least 5, or in some embodiments, at least 6, and up to 15 amino acid residue deletion at the amino terminus, or at the carboxyl terminus, or both so long as the peptide retains anti-inflammatory activity.

According to another aspect, the present invention provides a pharmaceutical composition comprising as an active agent an isolated polypeptide as herein described, and a pharmaceutically acceptable carrier for use in treating inflammation in a subject..

The pharmaceutical composition may comprise as an active agent, an isolated peptide consisting of an amino acid sequence, as set forth in any one of SEQ ID NOs:2 to SEQ ID NO:4, and a pharmaceutically acceptable carrier.

According to some embodiments, the pharmaceutical composition is formulated in a form selected from the group consisting of a solution, suspension, emulsion, powder, cream, lotion, gel, foam, spray, or aerosol.

The present specification also discloses a method for treating inflammation, for example, inflammation and/or inflammation caused by severe infections, the method comprising administering to a subject in need of such treatment a therapeutically effective amount of a an isolated peptide comprising the amino acid sequence as set forth in SEQ ID NO:1, or a biologically active analog, derivative or fragment thereof, or a pharmaceutical composition comprising a polypeptide as herein described as an active agent and a pharmaceutically acceptable carrier. Preferably, the subject is a human.

The pharmaceutical composition for use in the treatment of inflammation comprises as an active agent an isolated polypeptide as set forth in any one of SEQ ID NOs:1 to SEQ ID NO:4.

The route of administering the pharmaceutical composition may be selected from the group consisting of intravenous, subcutaneous, intramuscular, intraperitoneal, oral, nasal, aerosol, rectal, vaginal, and/or directly or adjacent to a damaged tissue. According to a certain embodiment, the route of administering the pharmaceutical composition is by intraperitoneal administration.

Due to their anti-inflammatory and immuno-modulating properties, the pharmaceutical compositions to be used according to the present invention are useful for treating a diverse group of indications having an inflammatory or autoimmune mechanism involved in their etiology or pathogenesis. According to some embodiments, the disease or condition is selected from the group consisting of inflammatory diseases, autoimmune diseases, degenerative neurological diseases, degenerative muscle diseases, wounds, hypersensitivity, infectious diseases, diseases associated with graft transplantation, allergic diseases, musculo-skeletal inflammations, and sepsis

According to additional embodiments, the inflammatory or autoimmune disease is selected from the group consisting of systemic lupus erythematosus (SLE), multiple sclerosis, arthritis including rheumatoid arthritis, inflammatory bowel disease (Crohn's disease), asthma, allergy, chronic bronchitis, sepsis, and psoriasis. According to certain exemplary embodiments, the disease to be treated is systemic lupus erythematosus (SLE), multiple sclerosis (MS) or psoriasis.

According to additional embodiments, the degenerative neurological disease is selected from the group consisting of amyotrophic lateral sclerosis, Parkinson's disease, and Alzheimer's disease.

According to a still further aspect, the present disclosure relates to a method for protecting against or treating a T- or B-cell mediated disease comprising administering to the subject in need of such treatment a therapeutically effective amount of a pharmaceutical composition according to the principles of the present invention, and a pharmaceutically acceptable carrier. According to some embodiments, the pharmaceutical composition to be administered for protecting against or treating a T- or B-cell mediated disease in a subject comprises a peptide of SEQ ID NO:1. Preferably, the subject is human.

According to some embodiments, T- or B-cell mediated disease that can be treated by a pharmaceutical composition of the invention is selected from the group consisting of psoriasis; allergy; T or B cell lymphomas and other malignancies; graft versus host disease; bronchitis; asthma; allergy; autoimmunity; sarcoidosis; bone marrow depression; bone marrow stimulation; depression or other mood or psychotic disorders; sepsis; myasthenia gravis (MG); Parkinson's disease; skin disorders and irritation; arthritis; multiple sclerosis; neurodegenerative disorders (amyotrophic lateral sclerosis, chorea, Alzheimer disease); atherosclerosis; fibrosis; pain; chronic or acute inflammation. The polypeptides and pharmaceutical compositions of the present invention for the use in the treatment of the aforesaid diseases can be used in combination therapy with standard medicaments for the diseases listed herein above.

The present disclosure also provides use of the polypeptides of the invention in the preparation of a medicament in the treatment of a disease or condition attributable to inflammatory processes. A specific example of such a diseases is sepsis.

It should be appreciated that alpha-1-antitrypsin or any known fragment thereof are excluded from the polypeptides of the present invention, but are disclosed and claimed for the novel uses discloses herein.

### BRIEF DESCRIPTION OF THE FIGURES

FIG. **1** shows the effect of UBE on Carrageenan-induced hind paw swelling.
FIG. **2** shows the effect of UBE on the neurological score of mice having experimental autoimmune encephalitis (EAE).
FIG. **3** shows the effect of UBE derivatives on the neurological score of mice having experimental autoimmune encephalitis.
FIG. **4** shows the effect of UBE on proteinuria in MRL/lpr mice having spontaneous lupus-like syndrome.
FIG. **5** shows the effect of UBE on microhematuria in MRL/lpr mice having spontaneous lupus-like syndrome.
FIG. **6** shows the effect of UBE on anti-dsDNA antibodies in blood samples of MRL/lpr mice having spontaneous lupus-like syndrome.
FIG. **7** shows the effect of UBE on the pathological score of kidneys of MRL/lpr mice having spontaneous lupus-like syndrome.
FIG. **8** shows the effect of UBE on proteinuria in MRL/lpr mice having spontaneous lupus-like syndrome at advanced stage of the disease.
FIG. **9** shows the effect of UBE or UBE1 on joint swelling in arthritic mice.
FIG. **10** shows the effect of UBE on lymphocyte proliferation in culture.
FIG. **11** shows the effect of UBE on HaCaT keratinocyte proliferation.
FIG. **12** shows the effect of UBE on oxidative burst of peritoneal macrophages.
FIG. **13** shows the effects of UBE on LPS induced model of sepsis in mice.
FIG. **14** shows the effects of UBE in an LPS-induced model of sepsis in mice.
FIG. **15** shows the effects of UBE on an OVA-induced model of asthma in mice.

### DETAILED DESCRIPTION OF THE INVENTION

The present invention provides isolated polypeptides derived from the amino acid sequence at positions 378 to 413 of *Mus musculus* Alpha 1-antitrypsin (serine protease inhibitor, clade A, member 1c) for use in the treatment of conditions attributable to inflammatory processes.. The present invention provides pharmaceutical compositions comprising same for use in the treatment of conditions attributable to inflammatory processes.

Particularly, the present invention provides an isolated polypeptide termed UBE1 having the sequence YSMPPIVRFDHPFLFIIFEEHTQSPLFVGKVVDPTHK as set forth in SEQ ID NO:1 derived from amino acid residues 377 to 413 of *Mus Musculus* Alpha 1-antitrypsin for use in treating inflammation. The present invention also provides an isolated polypeptide termed UBE having the sequence SMPPIVRFDHPFLFIIFEEHTQSPLFVGKVVDPTHK as set forth in SEQ ID NO:2 derived from amino acid residues 378 to 413 of *Mus musculus* Alpha 1-antitrypsin (serine protease inhibitor, clade A, member 1c) for use in treating inflammation. The present invention further provides an isolated peptide termed UBE-N having the sequence MPPIVRFDHPFLFIIFEEHTQSPLFVGKVVDPTHK as set forth in SEQ ID NO:3 derived from amino acid residues 379 to 413 of *Mus musculus* Alpha 1-antitrypsin for use in treating inflammation. The present invention further provides an isolated peptide termed UBE-C having the sequence SMPPIVRFDHPFLFIIFEEHTQSPLFVGKVVDPTH as set forth in SEQ ID NO:4 derived from amino acid residues 378 to 412 of *Mus musculus* Alpha 1-antitrypsin for use in treating inflammation. The polypeptides to be used according to the present invention exert anti-inflammatory and/or anti-autoimmune activity, and are therefore useful for treating or protecting a subject against inflammatory conditions and/or diseases associated with autoimmune activity.

As exemplified herein below, administration of the peptide UBE and its derivatives resulted in pharmacological effects on animal models of SLE, multiple sclerosis (MS), arthritis, sepsis and asthma. The polypeptides to be used according to the present invention protected against renal dysfunction in mice having SLE as measured by proteinuria and hematuria and reduced serum anti dsDNA antibodies in these mice, hence improved animal survival. The polypeptides to be used according to the present invention ameliorated neurological symptoms in an animal model of multiple sclerosis (MS). The polypeptides to be used according to the invention improved survival of mice injected with LPS an accepted model for sepsis. The polypeptides to be used according to the present invention reduced some of the hallmarks of airway inflammation by diminishing the % eosinophil recovery in BAL in a murine experimental asthma model.

As described herein, the polypeptides to be used according to the present invention were also capable of suppressing proliferation of isolated T cells. The polypeptides to be used according to the present invention demonstrated anti-proliferative effects on cultured also on non-immune cells such as HaCaT keratinocytes. The polypeptides to be used according to the present invention are, therefore, useful for treating diseases such as inflammatory, autoimmune, and connective tissue diseases.

The term "polypeptide" as used herein refers to a linear series of natural, non-natural and/or chemically modified amino acid residues connected one to the other by peptide bonds. The amino acid residues are represented throughout the specification and claims by either one or three-letter codes, as is commonly known in the art. The term "isolated polypeptide" refers to a polypeptide which has been separated from polypeptides, proteins or other biological molecules which are present in a naturally occurring state.

According to the principles of the present invention the polypeptides for the use according to the present invention do not include the intact alpha-1 antitrypsin protein or any known fragments thereto.

The term "the polypeptides to be used according to the present invention" refers to the polypeptides disclosed herein as UBE (SEQ ID NO:2), UBE1 (SEQ ID NO1), UBE-N (SEQ ID NO:3), UBE-C (SEQ ID NO:4).

The polypeptides for the use according to the present invention can be produced by various methods known in the art, including recombinant production or synthetic production. Recombinant production can be achieved by the use of an isolated polynucleotide encoding the polypeptides of the present invention, the isolated polynucleotide operably linked to a promoter for the expression of the polynucleotide. Optionally, a signal polypeptide and a regulator of the promoter are added. The construct comprising the polynucleotide encoding the polypeptides for the use according to the present invention can be placed in a vector, such as a plasmid, virus or phage vector. The vector can be used to transfect or transform a host cell, e.g., a bacterial, yeast, insect, or mammalian cell. The vector can also be introduced into a transgenic animal such as, for example, a transgenic mouse.

Alternatively, the polypeptide can be produced synthetically. Synthetic production of polypeptides is well known in the art. The polypeptides for the use according to the present invention can be synthesized using standard direct polypeptide synthesis (see, for example, Bodanszky, 1984, Principles of Polypeptide Synthesis, Springer-Verlag, Heidelberg), such as via solid-phase synthesis (see, for example, Merrifield, 1963, J. Am. Chem. Soc. 85:2149-2154). Examples of solid phase polypeptide synthesis methods include, but are not limited to, the BOC method, which utilizes tert-butyloxcarbonyl as the α-amino protecting group, and the FMOC method, which utilizes 9-fluorenylmethyloxcarbonyl to protect the α-amino of the amino acid residues, both methods are well-known by those of skill in the art.

Alternatively, the polypeptide can be synthesized using standard solution methods (see, for example, Bodanszky, M., Principles of Peptide Synthesis, Springer-Verlag, 1984).

According to another aspect, the present disclosurerelates to an isolated polynucleotide sequence encoding the polypeptides to be used according to the present inventionThe term "polynucleotide" means a polymer of deoxyribonucleic acid (DNA), ribonucleic acid (RNA) or a combination thereof, which can be derived from any source, can be single- or double-stranded, and can optionally contain synthetic, non-natural, or altered nucleotides, which are capable of being incorporated into DNA or RNA polymers.

An "isolated polynucleotide" refers to a polynucleotide segment or fragment which has been separated from sequences which flank it in a naturally occurring state, e.g., a DNA fragment which has been removed from the sequences which are normally adjacent to the fragment, e.g., the sequences adjacent to the fragment in a genome in which it naturally occurs. The term also applies to polynucleotides, which have been substantially purified from other components, which naturally accompany the polynucleotide in the cell, e.g., RNA or DNA or proteins. The term therefore includes, for example, a recombinant DNA which is incorporated into a vector, into an autonomously replicating plasmid or virus, or into the genomic DNA of a prokaryote or eukaryote, or which exists as a separate molecule (e.g., as a cDNA or a genomic or cDNA fragment produced by PCR or restriction enzyme digestion) independent of other sequences. It also includes a recombinant DNA, which is part of a hybrid gene encoding additional polypeptide sequence, and RNA such as mRNA.

The term "encoding" refers to the inherent property of specific sequences of nucleotides in an isolated polynucleotide, such as a gene, a cDNA, or an mRNA, to serve as templates for synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a polypeptide or protein if transcription and translation of mRNA corresponding to that gene produces the polypeptide or protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the polypeptide or protein or other product of that gene or cDNA.

One who is skilled in the art will appreciate that more than one polynucleotide may encode any given polypeptide or protein in view of the degeneracy of the genetic code and the allowance of exceptions to classical base pairing in the third position of the codon, as given by the so-called "Wobble rules." It is intended that the present invention encompass polynucleotides that encode the polypeptides of the present invention as well as any derivative, analog, and fragment thereof.

A polynucleotide as disclosed can be expressed as a secreted polypeptide where the polypeptides as referred to herein are isolated from the medium in which the host cell containing the polynucleotide is grown, or the polynucleotide can be expressed as an intracellular polypeptide by deleting the leader or other polypeptides, in which case the polypeptides of the present invention, derivative, analog or fragment thereof is isolated from the host cells. The polypeptides to be used accordant to the present invention so isolated are then purified by standard protein purification methods known in the art.

The polypeptides to be used according to the present invention can also be provided to the tissue of interest by transferring an expression vector comprising an isolated polynucleotide encoding the polypeptides to cells associated with the tissue of interest. The cells produce the polypeptide such that it is suitably provided to the cells within the tissue to exert a biological activity such as, for example, to reduce or inhibit inflammatory processes within the tissue of interest.

The expression vector according to the principles of the present invention further comprises a promoter. In the context of the present invention, the promoter must be able to drive the expression of the polypeptide within the cells. Many viral promoters are appropriate for use in such an expression vector (e.g., retroviral ITRs, LTRs, immediate early viral promoters (IEp) (such as herpes virus IEp (e.g., ICP4-IEp and ICP0-IEp) and cytomegalovirus (CMV) IEp), and other viral promoters (e.g., late viral promoters, latency-active promoters (LAPs), Rous Sarcoma Virus (RSV) promoters, and Murine Leukemia Virus (MLV) promoters). Other suitable promoters are eukaryotic promoters, which contain enhancer sequences (e.g., the rabbit β-globin regulatory elements), constitutively active promoters (e.g., the β-actin promoter, etc.), signal and/or tissue specific promoters (e.g., inducible and/or repressible promoters, such as a promoter responsive to TNF or RU486, the metallothionine promoter, etc.), and tumor-specific promoters.

Within the expression vector, the polynucleotide encoding the polypeptides to be used according to the present invention, and the promoter are operably linked such that the promoter is able to drive the expression of the polynucleotide. As long as this operable linkage is maintained, the expression vector can include more than one gene, such as multiple genes separated by internal ribosome entry sites (IRES). Furthermore, the expression vector can optionally include other elements, such as splice sites, polyadenylation sequences, transcriptional regulatory elements (e.g., enhancers, silencers, etc.), or other sequences.

The expression vectors are introduced into the cells in a manner such that they are capable of expressing the isolated polynucleotide encoding the polypeptides of the present invention, a fragment, derivative or analog thereof contained therein. Any suitable vector can be so employed, many of which are known in the art. Examples of such vectors include naked DNA vectors (such as oligonucleotides or plasmids), viral vectors such as adeno-associated viral vectors (Berns et al., 1995, Ann. N.Y. Acad. Sci. 772:95-104), adenoviral vectors, herpes virus vectors (Fink et al., 1996, Ann. Rev. Neurosci. 19:265-287), packaged amplicons (Federoff et al., 1992, Proc. Natl. Acad. Sci. USA 89:1636-1640), papilloma virus vectors, picornavirus vectors, polyoma virus vectors, retroviral vectors, SV40 viral vectors, vaccinia virus vectors, and other vectors. Additionally, the vector can also include other genetic elements, such as, for example, genes encoding a selectable marker (e.g., β-gal or a marker conferring resistance to a toxin), a pharmacologically active protein, a transcription factor, or other biologically active substance.

Methods for manipulating a vector comprising an isolated polynucleotide are well known in the art (e.g., Sambrook et al., 1989, Molecular Cloning: A Laboratory Manual, 2d edition, Cold Spring Harbor Press) and include direct cloning, site specific recombination using recombinases, homologous recombination, and other suitable methods of constructing a recombinant vector. In this manner, an expression vector can be constructed such that it can be replicated in any desired cell, expressed in any desired cell, and can even become integrated into the genome of any desired cell.

The expression vector comprising the polynucleotide of interest is introduced into the cells by any means appropriate for the transfer of DNA into cells. Many such methods are well known in the art (e.g., Sambrook et al., supra; see also Watson et al., 1992, Recombinant DNA, Chapter 12, 2d edition, Scientific American Books). Thus, in the case of prokaryotic cells, vector introduction can be accomplished, for example, by electroporation, transformation, transduction, conjugation, or mobilization. For eukaryotic cells, vectors can be introduced through the use of, for example, electroporation, transfection, infection, DNA coated microprojectiles, or protoplast fusion. Examples of eukaryotic cells into which the expression vector can be introduced include, but are not limited to, ovum, stem cells, blastocytes, and the like.

Cells, into which the polynucleotide has been transferred under the control of an inducible promoter if necessary, can be used as transient transformants. Such cells themselves may then be transferred into a subject for therapeutic benefit therein. Thus, the cells can be transferred to a site in the subject such that the polypeptide of the invention is expressed therein and secreted therefrom and thus reduces or inhibits, for example, inflammatory processes so that the clinical condition of the subject is improved. Alternatively, particularly in the case of cells to which the vector has been added in vitro, the cells can first be subjected to several rounds of clonal selection (facilitated usually by the use of a selectable marker sequence in the vector) to select for stable transformants. Such stable transformants are then transferred to a subject, preferably a human, for therapeutic benefit therein.

Within the cells, the polynucleotide encoding the polypeptides to be used according to the present invention is expressed, and optionally is secreted. Successful expression of the polynucleotide can be assessed using standard molecular biology techniques (e.g., Northern hybridization, Western blotting, immunoprecipitation, enzyme immunoassay, etc.).

The polypeptides to be used according to the present invention produced by recombinant techniques can be purified so that the polypeptides will be substantially pure when administered to a subject. The term "substantially pure" refers to a compound, e.g., a polypeptide, which has been separated from components, which naturally accompany it. Typically, a polypeptide is substantially pure when at least 50%, preferably at least 75%, more preferably at least 90%, and most preferably at least 99% of the total material (by volume, by wet or dry weight, or by mole percent or mole fraction) in a sample is the polypeptide of interest. Purity can be measured by any appropriate method, e.g., in the case of polypeptides by HPLC analysis.

The present disclosure also relates to transgenic animals comprising an isolated polynucleotide encoding the polypeptides of the invention.

### Pharmaceutical compositions and administration routes

The present invention provides pharmaceutical compositions comprising as an active agent a therapeutically effective amount of a polypeptide as disclosed, and a pharmaceutically acceptable carrier for use in the treatment of inflammation.

The pharmaceutical compositions of the disclosure can be formulated in the form of a pharmaceutically acceptable salt of the polypeptides to be used according to the present invention. Pharmaceutically acceptable salts include those salts formed with free amino groups such as salts derived from non-toxic inorganic or organic acids such as hydrochloric, phosphoric, acetic, oxalic, tartaric acids, and the like, and those salts formed with free carboxyl groups such as salts derived from non-toxic inorganic or organic bases such as sodium, potassium, ammonium, calcium, ferric hydroxides, isopropylamine, triethylamine, 2-ethylamino ethanol, histidine, procaine, and the like.

The term "pharmaceutically acceptable" means suitable for administration to a subject, e.g., a human. For example, the term "pharmaceutically acceptable" can mean approved by a regulatory agency of the Federal or a state government or listed in the U. S. Pharmacopeia or other generally recognized pharmacopeia for use in animals, and more particularly in humans. The term "carrier" refers to a diluent, adjuvant, excipient, or vehicle with which the therapeutic compound is administered. Such pharmaceutical carriers can be sterile liquids, such as water and oils, including those of petroleum, animal, vegetable or synthetic origin, such as peanut oil, soybean oil, mineral oil, sesame oil and the like, polyethylene glycols, glycerin, propylene glycol or other synthetic solvents. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like. The composition, if desired, can also contain minor amounts of wetting or emulsifying agents, or pH buffering agents such as acetates, citrates or phosphates. Antibacterial agents such as benzyl alcohol or methyl parabens; antioxidants such as ascorbic acid or sodium bisulfite; and agents for the adjustment of tonicity such as sodium chloride or dextrose are also envisioned.

The compositions can take the form of solutions, suspensions, emulsions, tablets, pills, capsules, powders, gels, creams, ointments, foams, pastes, sustained-release formulations and the like. The compositions can be formulated as a suppository, with traditional binders and carriers such as triglycerides, microcrystalline cellulose, gum tragacanth or gelatin. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in: Remington's Pharmaceutical Sciences" by E.W. Martin. Such compositions will contain a therapeutically effective amount of a source of the polypeptides of the current invention, preferably in a substantially purified form, together with a suitable amount of carrier so as to provide the form for proper administration to the subject.

The amount of the polypeptides for use according to the current invention, which will be effective in the treatment of a particular disorder or condition will depend on the nature of the disorder or condition and on the particular polypeptide source, and can be determined by standard clinical techniques known to a person skilled in the art. In addition, in vitro assays may optionally be employed to help identify optimal dosage ranges. The precise dose to be employed in the formulation will also depend on the route of administration, and the nature of the disease or disorder, and should be decided according to the judgment of the practitioner and each patient's circumstances. Effective doses can be extrapolated from dose-response curves derived from in-vitro or in-vivo animal model test bioassays or systems.

Depending on the location of the tissue of interest, a polypeptide to be used according to the current invention can be supplied in any manner suitable for the provision of the polypeptide to cells within the tissue of interest. Thus, for example, a composition containing the polypeptides to be used according to the current invention can be introduced, for example, into the systemic circulation, which will distribute the polypeptides to the tissue of interest. Alternatively, a composition containing a polypeptide to be used according to the current invention can be applied topically to the tissue of interest (e.g., injected, or pumped as a continuous infusion, or as a bolus within a tissue, applied to all or a portion of the surface of the skin, etc.).

The route of administration of the pharmaceutical composition will depend on the disease or condition to be treated. Suitable routes of administration include, but are not limited to, parenteral injections, e.g., intradermal, intravenous, intramuscular, intralesional, subcutaneous, intrathecal, and any other mode of injection as known in the art. Although the bioavailability of polypeptides administered by other routes can be lower than when administered via parenteral injection, by using appropriate formulations it is envisaged that it will be possible to administer the compositions of the invention via oral, transdermal, rectal, vaginal, topical, nasal, inhalation and ocular modes of treatment. In addition, it may be desirable to introduce the pharmaceutical compositions to be used according to the invention into the central nervous system by any suitable route, including intraventricular and intrathecal injection; intraventricular injection may be facilitated by an intraventricular catheter, for example, attached to a reservoir. Pulmonary administration can also be employed, e.g., by use of an inhaler or nebulizer.

It may be desirable to administer the pharmaceutical composition to be used according to the invention locally to the area in need of treatment; this can be achieved by, for example, and not by way of limitation, local infusion, topical application, by injection, by means of a catheter, by means of a suppository, or by means of an implant, said implant being of a porous, non-porous, or gelatinous material. Administration can be by direct injection e.g., via a syringe, at the site of a damaged tissue.

For topical application, the polypeptides to be used according to the current invention can be combined with a pharmaceutically acceptable carrier so that an effective dosage is delivered, based on the desired activity. According to an exemplary embodiment, the polypeptide to be used according to the invention is applied to the skin for treatment of diseases such as psoriasis. The carrier can be in the form of, for example, and not by way of limitation, an ointment, cream, gel, paste, foam, aerosol, suppository, pad or gelled stick.

For oral applications, the pharmaceutical composition may be in the form of tablets or capsules, which can contain any of the following ingredients, or compounds of a similar nature: a binder such as microcrystalline cellulose, gum tragacanth or gelatin; an excipient such as starch or lactose; a disintegrating agent such as alginic acid, Primogel, or corn starch; a lubricant such as magnesium stearate; or a glidant such as colloidal silicon dioxide. When the dosage unit form is a capsule, it can contain, in addition to the ingredients of the above type, a liquid carrier such as fatty oil. In addition, dosage unit forms can contain various other materials which modify the physical form of the dosage unit, for example, coatings of sugar, shellac, or other enteric agents. The tablets of the invention can further be film coated.

The polypeptides to be used according to the current invention can be delivered in a controlled release system. Thus, an infusion pump can be used to administer the polypeptide such as the one that is used, for example, for delivering insulin or chemotherapy to specific organs or tumors. The polypeptide to be used according to the invention may be administered in combination with a biodegradable, biocompatible polymeric implant, which releases the polypeptide over a controlled period of time at a selected site. Examples of preferred polymeric materials include, but are not limited to, polyanhydrides, polyorthoesters, polyglycolic acid, polylactic acid, polyethylene vinyl acetate, copolymers and blends thereof (See, Medical applications of controlled release, Langer and Wise (eds.), 1974, CRC Pres., Boca Raton, Fla.). A controlled release system can be placed in proximity to a therapeutic target, thus requiring only a fraction of the systemic dose.

The compositions to be used according to the present invention can be placed on a stent.

### Uses of UBE polypeptides

The present disclosure relates to a method for preventing or treating diseases or disorders attributable to inflammatory processes in a subject. The present disclosure also relates to a method for treating a disease or condition attributable to autoimmune processes in a subject. The present disclosure further relates to a method for treating T- or B-cell mediated disease in a subject.

The present disclosure relates to a method for treating inflammation in a subject in need thereof, said method comprising administering to said subject a therapeutically effective amount of a polypeptide as herein described.

In some embodiments, the inflammation is associated with an autoimmune disease, a wound, hypersensitivity, a degenerative neurological disease, a degenerative muscle disease, an infectious disease, a graft transplantation, an allergy, a musculo-skeletal condition, or sepsis. In some embodiments, the inflammation is associated with systemic lupus erythematosus (SLE), multiple sclerosis, arthritis, asthma, allergy, inflammatory bowel disease, Crohn's disease, or psoriasis. In some embodiments the subject suffers from systemic lupus erythematosus (SLE) and in some embodiments, the subject suffers from multiple sclerosis. In some embodiments, the subject suffers from psoriasis.

In some embodiments, the inflammation is associated with Parkinson's disease, amyotrophic lateral sclerosis, or Alzheimer's disease. In some embodiments, the inflammation is associated with sepsis.

The methods disclosed herein comprise the step of administering to a subject in need of such treatment a pharmaceutical composition comprising as an active agent a therapeutically effective amount of a polypeptide to be used according to the current invention and a pharmaceutically acceptable carrier. According to a certain exemplary embodiment, the polypeptide is UBE polypeptide of SEQ ID NO:1. The polypeptide may be UBE1 polypeptide of SEQ ID NO:2. The polypeptide may be UBE-N polypeptide of SEQ ID NO:3. The polypeptide may also be UBE-C polypeptide of SEQ ID NO:4.

A "therapeutically effective amount" of the polypeptide to be used according to the current invention is that amount of the polypeptide source which is sufficient to provide a beneficial effect to the subject to which the source is administered. More specifically, a therapeutically effective amount means an amount of the source effective to prevent, alleviate or ameliorate tissue damage or symptoms of a disease of the subject being treated.

As the polypeptides for use according to the invention have proven to be particularly efficacious against inflammatory processes associated with systemic lupus erythematosus (SLE), multiple sclerosis, arthritis and sepsis. The polypeptides for use according to the invention are therefore very useful as bona fide anti-inflammatory or immuno-modulating agents.

As anti-inflammatory or immuno-modulating agents, the polypeptides for use according to the invention are expected to be efficacious in all diseases, disorders, or conditions that involve inflammation or inflammatory activity. Therefore, this invention relates to the protective effect of the polypeptide for the use according to the current invention against all disorders or diseases that are related to or involve inflammation.

Inflammatory diseases that can be treated by the polypeptide for use according to the current invention include autoimmune diseases including, but not limited to, systemic lupus erythematosus (SLE), multiple sclerosis (MS), arthritis including rheumatoid arthritis, asthma, allergy, chronic bronchitis, inflammatory bowel disease (Crohn's disease), psoriasis, and sepsis.

Inflammation is also associated with chronic neurological degenerative diseases and muscle degenerative diseases. The degenerative diseases include, but are not limited to, multiple sclerosis, Alzheimer's disease, Parkinson's disease, myasthenia gravis, muscle dystrophy, and amyotrophic lateral sclerosis.

Inflammation is also associated with hypersensitivity. Hypersensitivity includes, but is not limited to, immediate hypersensitivity, antibody mediated hypersensitivity, immune complex mediated hypersensitivity, T lymphocyte mediated hypersensitivity and delayed type hypersensitivity.

Inflammation is also associated with an infectious disease. Infections-induced inflammations that can be treated with the pharmaceutical compositions of the invention include, but are not limited to those induced by viral diseases, bacterial diseases, protozoan diseases, parasitic diseases, fungal diseases, and mycoplasma diseases. Thus, the compositions comprising the polypeptide source of the invention can be used as cosmetics to eliminate skin inflammatory responses associated with infections

Inflammation can also be associated with transplantation of a graft, such as, for example, in conditions of graft rejection.

Inflammation can also be associated with an allergic disease and with musculo-skeletal inflammation. The musculo-skeletal inflammation is selected from the group consisting of arthritis, muscle inflammation, myositis, a tendon inflammation, tendinitis, a ligament inflammation, a cartilage inflammation, a joint inflammation, a synovial inflammation, carpal tunnel syndrome and a bone inflammation.

The polypeptides to be used according to the invention are useful for protecting against or treating T- or B-cell mediated disease. T- or B-cell mediated diseases include, but are not limited to, psoriasis; allergy; T or B cell lymphomas and other malignancies; graft versus host disease; prevention of transplant rejection; bronchitis; asthma; allergy; autoimmunity; sarcoidosis; bone marrow depression; bone marrow stimulation; sepsis; myasthenia gravis (MG); Parkinson's disease; skin disorders and irritation; arthritis; multiple sclerosis; neurodegenerative disorders (amyotrophic lateral sclerosis, chorea, Alzheimer disease); atherosclerosis; fibrosis; pain; chronic or acute inflammation.

The protective effect of the polypeptides for use according to the invention can be achieved by prophylactic treatment. The protective effect can also be achieved by post-exposure treatment with the polypeptide. Similarly, the protective effect of the polypeptides is achieved against inflammatory processes as exemplified herein below.

The term "protecting" relate to reduction of degree of lesion or biological damage as measured by gross pathology or histopathological evaluation, subjective burning sensation or other accepted parameters for tissue damage, lesion, discomfort and pain.

The pharmaceutical compositions to be used according to the invention can be used for accelerated healing of or prevention of development of wounds including decubitus, ulcers (also induced by drugs), internal and external wounds, abscesses and various bleedings.

The pharmaceutical compositions to be used according to the invention are useful for treatment or protection against tissue damage including, but not limited to, neuronal, neurological, skin, hepatic, nephrologic, urologic, cardiac, pulmonary, gastrointestinal, lower and upper airways, visual, audiologic, spleen, bone, and muscle damage. Treatment or protection against tissue damage can be accomplished in the fetus, newborn, child, adolescent as well as in adults and old persons, whether the condition or disorder to be treated is spontaneous, of traumatic etiology or as a congenital defect.

It will be understood that the pharmaceutical compositions for use according to the present invention can comprise the polypeptides to be used according to the current invention or all possible combinations of two or more of these polypeptides Thus, the pharmaceutical compositions can comprise one or more isolated polynucleotides, one or more expression vectors, or one or more host cells or any combination thereof, according to the principles of the present invention.

Determination of a therapeutically effective amount of a polypeptide is well within the capability of those skilled in the art, especially in light of the detailed disclosure provided herein.

Toxicity and therapeutic efficacy of the polypeptides described herein can be determined by standard pharmaceutical procedures in cell cultures or experimental animals, e.g., by determining the IC₅₀ (the concentration which provides 50% inhibition) and the LD₅₀ (lethal dose causing death in 50 % of the tested animals) for a subject compound. The data obtained from these cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See, for example, Fingl et al., 1975, in The Pharmacological Basis of Therapeutics, Ch. 1 p.1).

Depending on the severity and responsiveness of the condition to be treated, dosing can also be a single administration of a slow release composition, with course of treatment lasting from several days to several weeks or until cure is effected or diminution of the disease state is achieved.

The amount of a composition to be administered will, of course, depend on the subject being treated, the severity of the affliction, the manner of administration, the judgment of the prescribing physician, and all other relevant factors. Determination of the exact dose to be administered is conducted by methods known to a person of skill in the art.

It is further understood that the polypeptides for use according to the invention can be formulated or administered together with additional active ingredients as required to treat the condition of the patient.

It should be understood that in addition to the ingredients particularly mentioned above the formulations of this invention may include other agents or excipients conventional in the art having regard to the type of formulation in question, for example those suitable for oral administration may include flavoring agents.

For administration to mammals, and particularly humans, it is expected that in the case of medications, the physician or other qualified healthcare provider may determine the actual dosage and duration of treatment, which will be most suitable for an individual and can vary with the age, weight and response of the particular individual. It will be appreciated that in the case of non-prescription (e.g. "over-the-counter") medications, foods, food products, food supplements, cosmetic and personal care compositions, the amount may be determined at the discretion of the user, optionally with guidance from the labeling or from an appropriate health care provider or other advisor.

### EXAMPLE 1

### Effect Of UBE On Carrageenan-Induced Hind Paw Swelling

Male CD1 mice were intravenously (i.v.) injected with the indicated doses of the UBE polypeptide having the sequence SMPPIVRFDHPFLFIIFEEHTQSPLFVGKVVDPTHK (SEQ ID NO:2). Thirty minutes thereafter, each animal was injected with carrageenan (50 µl of 3 mg/ml into each hindpaw). The animals were evaluated for the difference between the degree of swelling (mm) after 3 hours and prior to carrageenan injection.

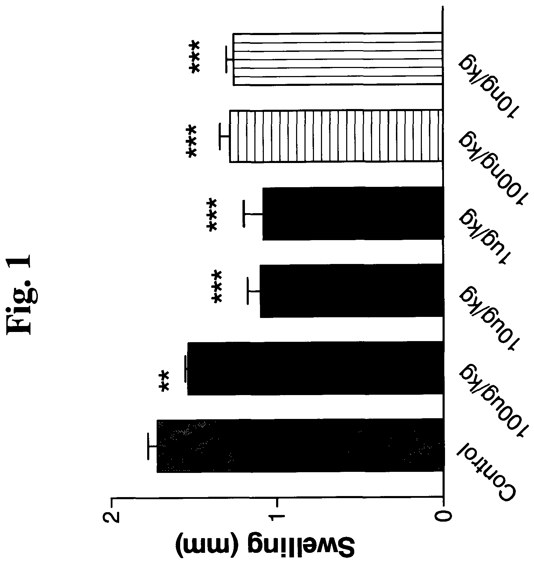

FIG. **1** shows a significant reduction in carrageenan-induced inflammation following i.p. administration of UBE.

### EXAMPLE 2

### Effect Of UBE On Experimental Autoimmune Encephalitis (EAE)

Female C57BL/6 mice were injected subcutaneously into 4 sites on the back, adjacent to each of the forelimbs and hindlimbs (total amount 200µl) with a myelin oligodendritic glycoprotein (MOG) 35-55 fragment emulsified with complete Freund's adjuvant. Thereafter, each animal was i.p. injected with pertusis toxin (PTX; 200 ng/mouse) in PBS and an additional PTX injection was repeated 2 days later, which is a standard protocol for initiating experimental autoimmune encephalomyelitis (EAE) in mice. UBE polypeptide (SEQ ID NO:2) was intraperitoneally (i.p.) injected (0.3 µg/kg, thrice a week) five days after MOG immunization (day of onset of symptoms). The animals were evaluated for neurological score from 0 (no effect) to 6 (severe neurological symptoms including paralysis). Results are the mean±SE of neurological score (sum of all scores divided by the number of animals in each experimental group) at each of the indicated time intervals after MOG injection.

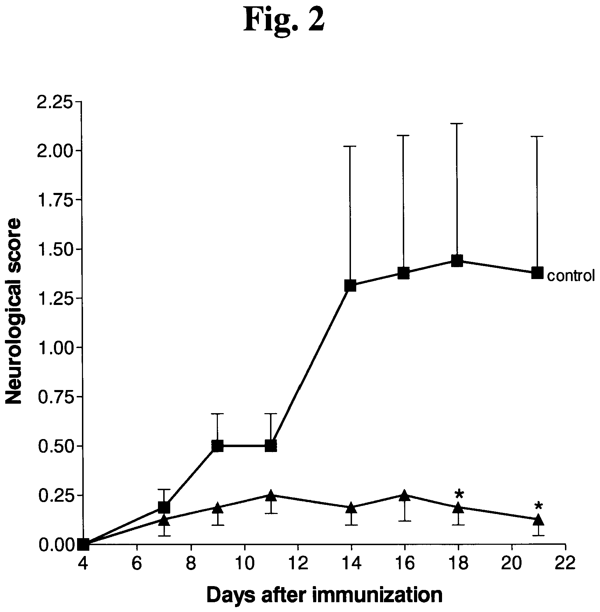

FIG. 2 shows that UBE when i.p. administered to EAE mice reduced the neurological symptoms associated with the disease.

### EXAMPLE 3

### Effect Of UBE Derivatives On Experimental Autoimmune Encephalitis

Female C57BL/6 mice were injected subcutaneously into 4 sites on the back, adjacent to each of the forelimbs and hindlimbs (total amount 200 µl) with a myelin oligodendritic glycoprotein (MOG) 35-55 fragment emulsified with complete Freund's adjuvant. Thereafter, each animal was i.p. injected with pertusis toxin (PTX; 200 ng/mouse) in PBS and an additional PTX injection was repeated 2 days latter. Four days after MOG immunization, each mouse received i.p. injections (1 µg/kg, thrice a week) of each of the following polypeptides:
SMPPIVRFDHPFLFIIFEEHTQSPLFVGKVVDPTHK (SEQ ID NO:2; designated UBE);
YSMPPIVRFDHPFLFIIFEEHTQSPLFVGKVVDPTHK (SEQ ID NO:1; designated UBE1);
MPPIVRFDHPFLFIIFEEHTQSPLFVGKVVDPTHK (SEQ IS NO:3; designated UBE-N); and
SMPPIVRFDHPFLFIIFEEHTQSPLFVGKVVDPTH (SEQ ID NO:4; designated UBE-C).

The animals were evaluated for neurological score from 0 (no effect) to 6 (severe neurological symptoms including paralysis). Results are the mean±SE of neurological score (sum of all scores divided by the number of animals in each experimental group) at each of the indicated time intervals after immunization.

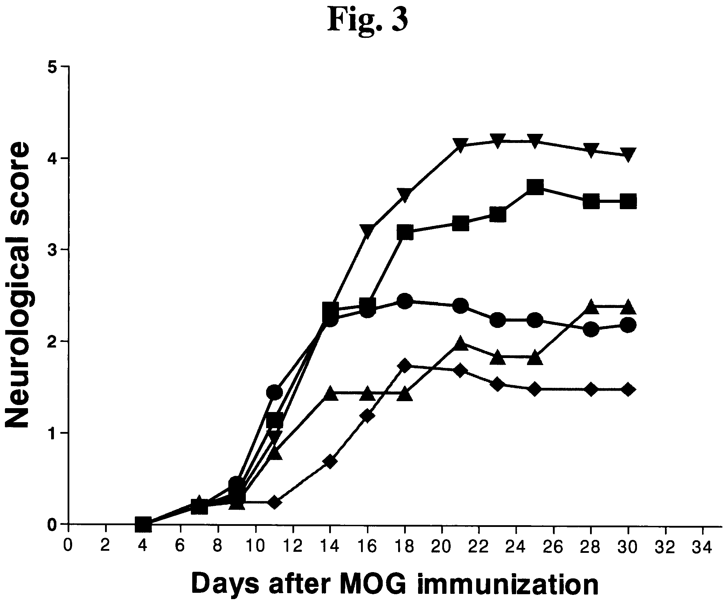

FIG. **3** shows that UBE, UBE-N, or UBE-C when i.p. administered to EAE mice reduced the neurological symptoms associated with the disease.

### EXAMPLE 4

### Effect Of UBE On Systemic Lupus Erythematosus

An animal model for Systemic Lupus Erythematosus was used to determine the effect of UBE on the manifestations of this disease.

MRL/lpr mice develop a lupus-like syndrome spontaneously. UBE (SEQ ID NO:2) (1 µg/kg) was administered to female MRL/lpr mice thrice a week, starting at age of 12 weeks. Urine was obtained by spontaneous urination and the levels of protein (mg/dl) and erythrocytes (cells/dl) in the urine were evaluated throughout the treatment period (4 weeks) using commercial sticks.

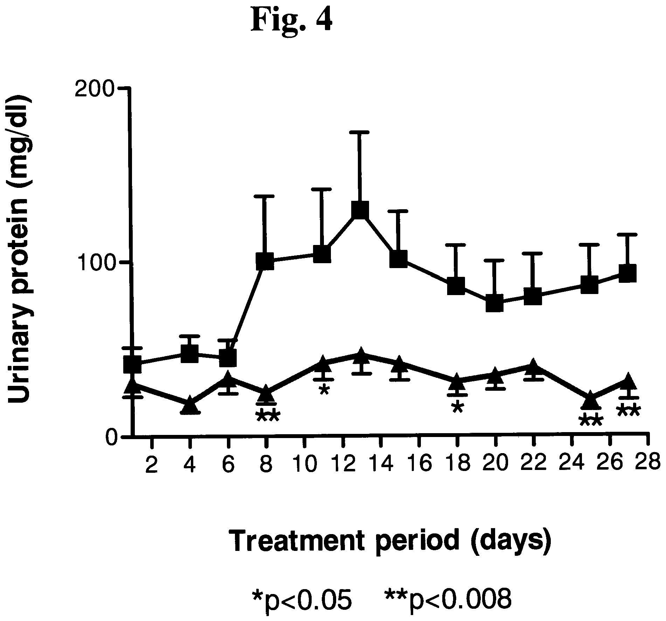

FIG. **4** shows a significant reduction in proteinuria following UBE administration to MRL/lpr mice.

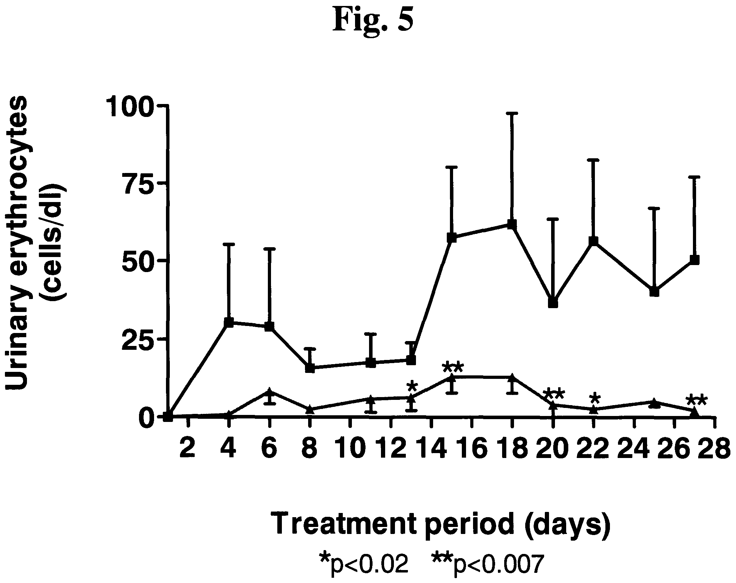

FIG. **5** shows a significant reduction in urinary microhematuria following UBE administration to MRL/lpr mice.

In order to determine the levels of anti-dsDNA antibodies, blood was obtained by cardiac puncture of the MRL/lpr mice after 4 weeks of UBE treatment, and the serum levels of anti-dsDNA antibodies were evaluated using a commercial ELISA kit.

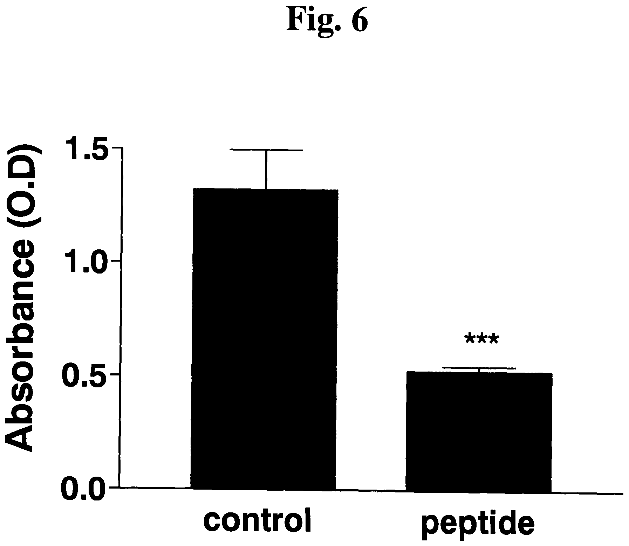

FIG. **6** shows a significant reduction in anti-dsDNA antibodies in the blood levels of MRL/lpr mice after UBE administration.

Quantitative histopathology analysis of the kidneys was also performed. The kidneys of the mice after 4 weeks of UBE treatment were removed, fixed in formalin, sliced, and stained by H&E and by periodic acid shift stain. The quantitative histopathology analysis was conducted on the glomeruli and tubulointerstitial areas.

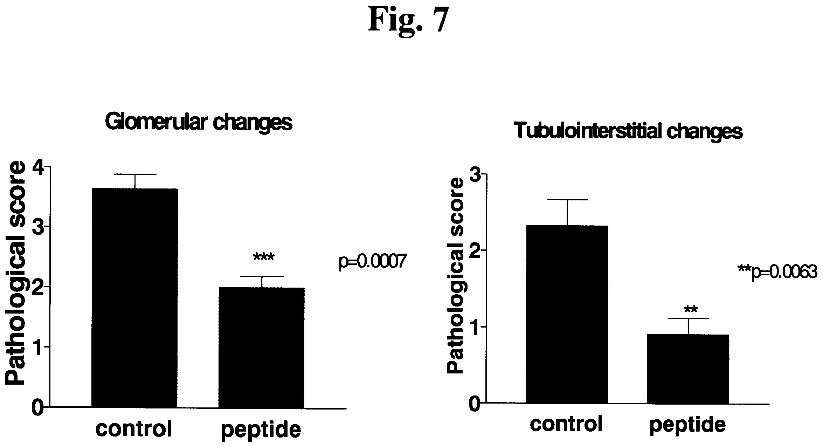

FIG. **7** shows a significant reduction in the pathological score of both the glomeruli and tubulointerstitial areas following UBE administration to MRL/lpr mice.

### EXAMPLE 5

### Effect of UBE at Advanced Stage of Systemic Lupus Erythematosus

UBE (SEQ ID NO: 2; 1µg/kg) was administered to male MRL/lpr mice thrice a week, starting at age of 22 weeks. At this age the mice are acutely affected and show a high level of proteinuria. Urine was obtained by spontaneous urination and the animals' urinary protein levels (mg/dl) were evaluated throughout the treatment period (22 days) using a commercial stick.

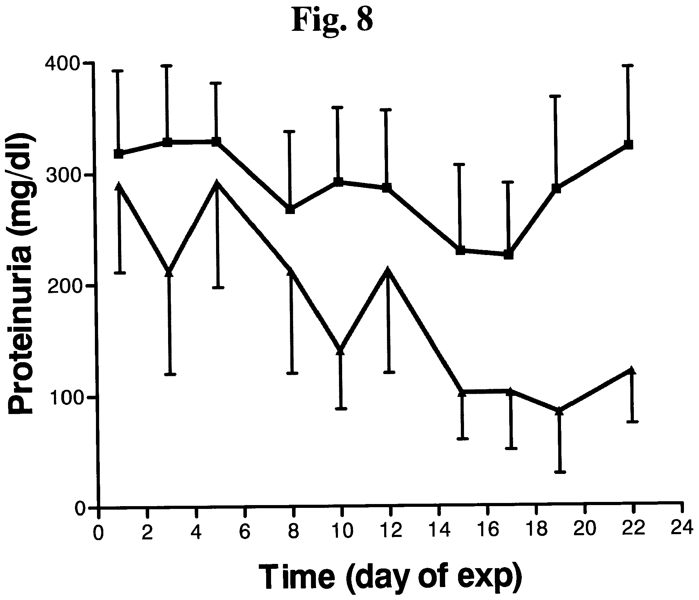

FIG. **8** shows a significant reduction in proteinuria following UBE administration to MRL/lpr mice.

### EXAMPLE 6

### Effect of Administration of UBE Or UBE1 on Arthritic Mice

Chick collagen type II (2.0 mg) was incubated overnight in 0.01 M acetic acid (0.9 ml) at 4° C. The resulting solution was emulsified with equal volume (1.0 ml) of incomplete Freund's Adjuvant containing 5.0 mg heat-killed Mycobacterium tuberculosis. Thereafter, the animal was injected intradermally in the tail base with 100 µl of the emulsion and an additional immunization was repeated 21 days later. Five days later, UBE or UBE1 polypeptide were administered (1µg/kg thrice a week). The animals were evaluated for the difference in joint thickness (mm) between the indicated time intervals and prior to immunization.

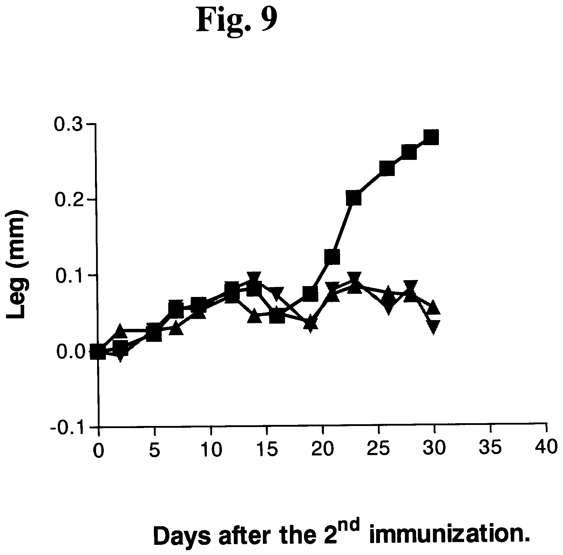

FIG. **9** shows a reduction in joint swelling in both UBE- and UBE1-treated mice.

### EXAMPLE 7

### Effect of UBE on Lymphocyte Proliferation In Vitro

T-lymphocytes were harvested from lymph nodes of female C57B1/6 mice by standard methodology and seeded (1.3x10⁶ cells/ml) in 96 well plate (100µl). Thereafter, UBE was added to the culture at the indicated concentrations and incubated for 96 hours (6% CO₂, 37°C). The medium containing the polypeptide was replaced after 48 hours of incubation. Viable cells (trypan blue staining) were counted a light microscope.

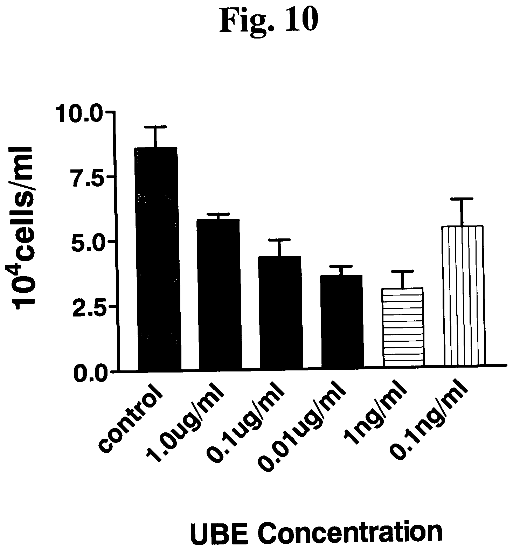

Fig. **10** shows a maximal effect of UBE on T cell proliferation at a concentration of 1 ng/ml.

### EXAMPLE 8

### Effect of UBE on HaCaT Keratinocyte Proliferation

Cells (1.6x10⁴/ml) were seeded (100 µl) in culture medium in 96 well plate. Twenty four hours later UBE was added at the indicated concentrations. Cell viability was determined by the MTT assay.

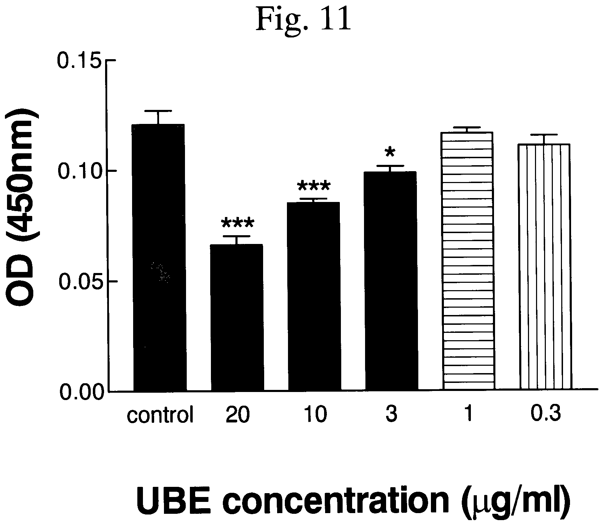

Fig. **11** demonstrates the anti-proliferative effect of UBE on cultured keratinocytes.

### EXAMPLE 9

### Effect of UBE on Oxidative Burst of Peritoneal Macrophages

Macrophages were harvested by peritoneal lavage of female C57BL/6 mice i.p. injected with thioglycolate. 0.2 ml cells (3x10⁵/ml), luminol (200 µM), horseradish peroxidase (1.0 unit/ml) and UBE were suspended in Hank's balanced salt solution (HBSS) (final concentrations of the polypeptide in the reaction mixture are indicated in the figure). The reaction started by the addition of 1µM phorbol myristate acetate. The reaction was performed in a 96-well plate. Luminescence was measured by Tecan spectrofluoro Plus.

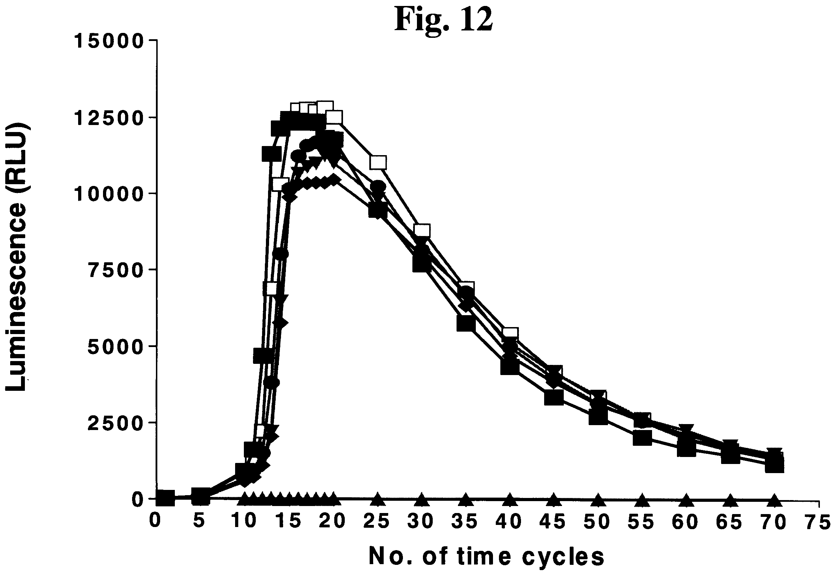

Fig **12** shows that 1µg/ml UBE suppressed oxidative burst of peritoneal macrophages.

### EXAMPLE 10

### Effect of UBE on LPS Induced Sepsis in Mice

Female C57B1/6 mice 7-8 weeks of age were injected i.p with LPS 1.2mg/mouse. Thereafter (within 1 hour) UBE polypeptide as depicted by SEQ ID NO: 2 was injected i.v. (100 µl) at different doses; control group received PBS. Survival of mice was monitored. n=10 for control group, n=9 for each UBE-treated group.

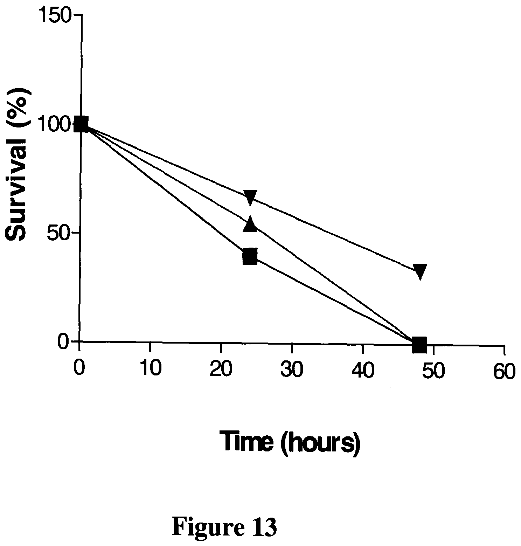

The results are shown in Figure 13. Injection of UBE reduced by 33% the LPS-induced mortality as observed 48 hours after LPS stimulation.

In a second protocol, mice were injected i.p. with LPS (dissolved in saline solution) at a dose of 1.2mg/mouse, followed by i.p. injection of UBE at a dose of 1.0 or10.0µg/kg and UBE treatment was repeated after 24 hours (n=4 for UBE-treated groups and n=5 for controls).

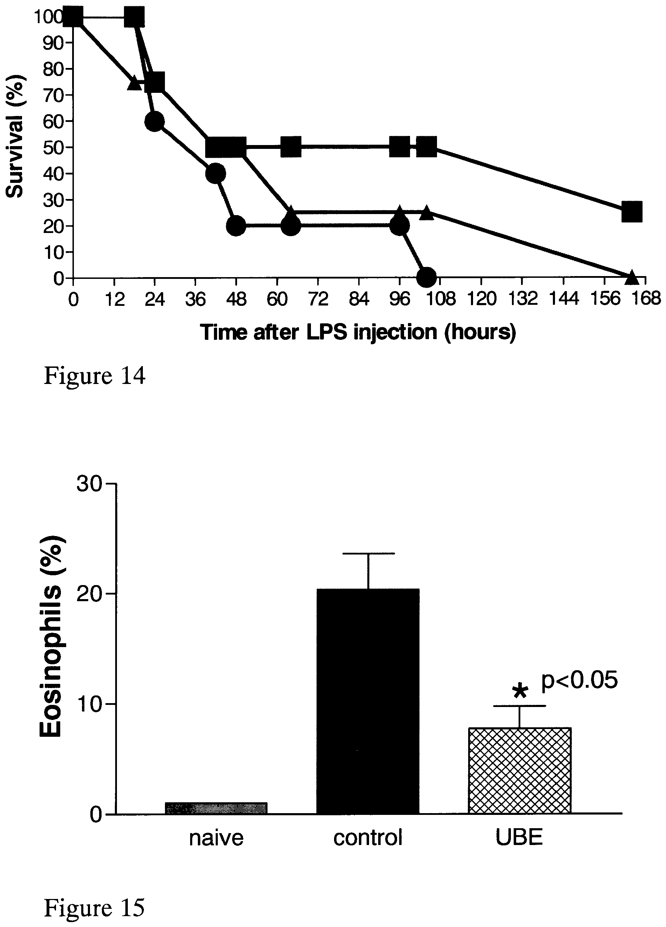

Figure 14 demonstrates that treatment with both 1.0 µg/kg and 10 µg/kg provided for survival of approximately 50% of the mice treated, whereas only 20% of controls survived to 50 hours post LPS injection. Both UBE dosages significantly prolonged survival in mice, as compared to controls.

### EXAMPLE 11

### Effect of UBE on Ovalbumin-Induced Asthma in Mice

20ul of 5% Ovalbumin (OVA) was added to 8.75 ml of sterile saline, which was then added stepwise to a solution of 1.25 ml of 40 mg/ml ImjectAlum (Pierce), and subjected to rocking at room temperature for 30-60 minutes, to facilitate optimal alum absorption to the Ovalbumin (Alum/Ova). Female Balb/C mice were then injected with 200ul of Alum/Ova i.p, according to the following protocol:

On Days 0 and 14, mice were injected i.p with OVA/Alum.

On day 24 mice were injected intranasally (i.n.) with. UBE/saline (50ng/50µl/mouse, 25µl to each nostril) and then after one hour, mice were injected i.n with OVA (50µg/50µl/mouse, 25µl to each nostril).

On Day 27, mice were injected i.n. UBE/saline (50ng/50µl/mouse, 25µl to each nostril) and then 1 hour subsequently were again injected i.n. with OVA (50µg/50µl/mouse). Mice were then sacrificed on day 29, and bronchoalveolar fluid was obtained by lung lavage (1ml of 10% FCS-heat inactivated in PBS), and the percent of eosinophils seen in lavage fluid versus white cell count was ascertained by micrscopic evaluation of smears.

Figure 15 demonstrates a dramatic reduction in eosinophil concentration in lavage fluid of mice treated with UBE as compared to controls, and the difference was statistically significant, as determined by the Student's t-test.

## Claims

1. An isolated polypeptide consisting of an amino acid sequence selected from the group consisting of SEQ ID NO: 1, SEQ ID NO: 2, SEQ ID NO: 3, and SEQ ID NO: 4, for use in treating inflammation in a subject.

2. An isolated polypeptide for use according to claim 1, wherein said inflammation is associated with an autoimmune disease, a wound, hypersensitivity, a degenerative neurological disease, a degenerative muscle disease, an infectious disease, a graft transplantation, an allergy, a musculoskeletal condition, or sepsis in said subject or said subject suffers from the same, systemic lupus erythematosus (SLE), multiple sclerosis, arthritis, asthma, inflammatory bowel disease, Crohn's disease, or psoriasis or said subject suffers from the same.

3. An expression vector comprising an isolated polynucleotide encoding a polypeptide consisting of an amino acid sequence selected from the group consisting of SEQ ID NO: 1, SEQ ID NO: 2, SEQ ID NO: 3, and SEQ ID NO: 4, for use in treating inflammation in a subject.

4. A host cell comprising an expression vector comprising a polynucleotide encoding a polypeptide consisting of an amino acid sequence selected from the group consisting of SEQ ID NO: 1, SEQ ID NO: 2, SEQ ID NO: 3, and SEQ ID NO: 4, for use in treating inflammation in a subject.

5. A pharmaceutical composition comprising as an active agent an isolated polynucleotide sequence encoding a polypeptide consisting of an amino acid sequence selected from the group consisting of SEQ ID NO: 1, SEQ ID NO: 2, SEQ ID NO: 3 and SEQ ID NO: 4, and a pharmaceutically acceptable carrier, for use in treating inflammation in a subject.

6. A pharmaceutical composition comprising as an active agent an expression vector comprising a polynucleotide encoding a polypeptide consisting of an amino acid sequence selected from the group consisting of SEQ ID NO: 1, SEQ ID NO: 2, SEQ ID NO: 3 and SEQ ID NO: 4, and a pharmaceutically acceptable carrier, for use in treating inflammation in a subj ect.

7. A pharmaceutical composition comprising as an active agent a host cell transfected with an expression vector comprising a polynucleotide encoding a polypeptide consisting of an amino acid sequence selected from the group consisting of SEQ ID NO: 1, SEQ ID NO: 2, SEQ ID NO: 3 and SEQ ID NO: 4, and a pharmaceutically acceptable carrier, for use in treating inflammation in a subject.

8. The pharmaceutical composition for use according to any of claims 5-7, wherein the pharmaceutical composition is formulated in a form selected from the group consisting of a solution, suspension, emulsion, powder, cream, lotion, gel, foam, spray, or aerosol.

## Patentansprüche

1. Isoliertes Polypeptid bestehend aus einer Aminosäuresequenz ausgewählt aus der Gruppe bestehend aus SEQ ID NR. 1, SEQ ID NR. 2, SEQ ID NR. 3, und SEQ ID NR. 4, zur Verwendung bei der Behandlung einer Entzündung in einem Individuum.

2. Isoliertes Polypeptid zur Verwendung nach Anspruch 1, worin die Entzündung assoziiert ist mit einer Autoimmunerkrankung, einer Wund-Hypersensitivität, einer degenerativen neurologischen Erkrankung, einer degenerativen Muskel-Erkrankung, einer infektiösen Erkrankung, einer Graft-Transplantation, einer Allergie, einem muskuloskeletalen Zustand, oder einer Sepsis in dem Individuum oder das Individuum leidet daran, systemischem Lupus Erythematosus (SLE), multipler Sklerose, Arthritis, Asthma, einer entzündlichen Darmerkrankung, Crohn's-Erkrankung oder Psoriasis oder das Individuum leidet daran.

3. Expressionsvektor, welcher ein isoliertes Polynukleotid enthält, das ein Polypeptid codiert, welches aus einer Aminosäuresequenz besteht ausgewählt aus der Gruppe bestehend aus SEQ ID NR. 1, SEQ ID NR. 2, SEQ ID NR. 3, und SEQ ID NR. 4, zur Verwendung bei der Behandlung einer Entzündung in einem Individuum.

4. Wirtszelle, welche einen Expressionsvektor enthält, der ein Polynukleotid umfasst, das ein Polypeptid codiert, welches aus einer Aminosäuresequenz besteht ausgewählt aus der Gruppe bestehend aus SEQ ID NR. 1, SEQ ID NR. 2, SEQ ID NR. 3, und SEQ ID NR. 4, zur Verwendung bei der Behandlung einer Entzündung in einem Individuum.

5. Pharmazeutische Zusammensetzung, welche als ein aktives Mittel eine isolierte Polynukleotidsequenz enthält, die ein Polypeptid umfasst, das aus einer Aminosäuresequenz besteht ausgewählt aus der Gruppe bestehend aus SEQ ID NR. 1, SEQ ID NR. 2, SEQ ID NR. 3 und SEQ ID NR. 4, und einen pharmazeutisch annehmbaren Träger zur Verwendung bei der Behandlung einer Entzündung in einem Individuum.

6. Pharmazeutische Zusammensetzung welche umfasst, als ein aktives Mittel einen Expressionsvektor, der ein Polynukleotid enthält, das ein Polypeptid codiert, welches aus einer Aminosäuresequenz besteht ausgewählt aus der Gruppe bestehend aus SEQ ID NR. 1, SEQ ID NR. 2, SEQ ID NR. 3 und SEQ ID NR. 4, und einen pharmazeutisch annehmbaren Träger zur Verwendung bei der Behandlung einer Entzündung in einem Individuum.

7. Pharmazeutische Zusammensetzung welche umfasst, als ein aktives Mittel eine Wirtszelle, die mit einem Expressionsvektor transfiziert ist, der ein Polynukleotid umfasst, das ein Polypeptid codiert, bestehend aus einer Aminosäuresequenz ausgewählt aus der Gruppe bestehend aus SEQ ID NR. 1, SEQ ID NR. 2, SEQ ID NR. 3 und SEQ ID NR. 4, und einen pharmazeutisch annehmbaren Träger zur Verwendung bei der Behandlung einer Entzündung in einem Individuum.

8. Pharmazeutische Zusammensetzung zur Verwendung nach einem der Ansprüche 5-7, worin die pharmazeutische Zusammensetzung in einer Form formuliert ist ausgewählt aus der Gruppe bestehend aus einer Lösung, Suspension, Emulsion, Pulver, Creme, Lotion, Gel, Schaum, Spray oder einem Aerosol.

## Revendications

1. Polypeptide isolé constitué d'une séquence d'acides aminés choisie dans le groupe constitué par les séquences SEQ ID NO : 1, SEQ ID NO : 2, SEQ ID NO : 3 et SEQ ID NO : 4, pour une utilisation dans le traitement d'une inflammation chez un sujet.

2. Polypeptide isolé pour une utilisation selon la revendication 1, ladite inflammation étant associée à une maladie auto-immune, à une plaie, à une hypersensibilité, à une maladie neurologique dégénérative, à une maladie musculaire dégénérative, à une maladie infectieuse, à une transplantation de greffe, à une allergie, à une affection musculo-squelettique ou à une sepsie chez ledit sujet ou dont ledit sujet souffre, au lupus érythémateux disséminé (SLE), à la sclérose en plaques, à l'arthrite, à l'asthme, à un maladie intestinale inflammatoire, à la maladie de Crohn ou au psoriasis ou dont ledit sujet souffre.

3. Vecteur d'expression comprenant un polynucléotide isolé codant pour un polypeptide constitué d'une séquence d'acides aminés choisie dans le groupe constitué par les séquences SEQ ID NO : 1, SEQ ID NO : 2, SEQ ID NO : 3 et SEQ ID NO : 4, pour une utilisation dans le traitement d'une inflammation chez un sujet.

4. Cellule hôte comprenant un vecteur d'expression comprenant un polynucléotide codant pour un polypeptide constitué d'une séquence d'acides aminés choisie dans le groupe constitué par les séquences SEQ ID NO : 1, SEQ ID NO : 2, SEQ ID NO : 3 et SEQ ID NO : 4, pour une utilisation dans le traitement d'une inflammation chez un sujet.

5. Composition pharmaceutique comprenant en tant que principe actif une séquence polynucléotidique isolée codant pour un polypeptide constitué d'une séquence d'acides aminés choisie dans le groupe constitué par les séquences SEQ ID NO : 1, SEQ ID NO : 2, SEQ ID NO : 3 et SEQ ID NO : 4 et un support pharmaceutiquement acceptable, pour une utilisation dans le traitement d'une inflammation chez un sujet.

6. Composition pharmaceutique comprenant en tant que principe actif un vecteur d'expression comprenant un polynucléotide codant pour un polypeptide constitué d'une séquence d'acides aminés choisie dans le groupe constitué par les séquences SEQ ID NO : 1, SEQ ID NO : 2, SEQ ID NO : 3 et SEQ ID NO : 4 et un support pharmaceutiquement acceptable, pour une utilisation dans le traitement d'une inflammation chez un sujet.

7. Composition pharmaceutique comprenant en tant que principe actif une cellule hôte transfectée avec un vecteur d'expression comprenant un polynucléotide codant pour un polypeptide constitué d'une séquence d'acides aminés choisie dans le groupe constitué par les séquences SEQ ID NO : 1, SEQ ID NO : 2, SEQ ID NO : 3 et SEQ ID NO : 4 et un support pharmaceutiquement acceptable, pour une utilisation dans le traitement d'une inflammation chez un sujet.

8. Composition pharmaceutique pour une utilisation selon l'une quelconque des revendications 5 à 7, la composition pharmaceutique étant formulée sous une forme choisie dans le groupe constitué par une solution, une suspension, une émulsion, une poudre, une crème, une lotion, un gel, une mousse, un spray ou un aérosol.


### SEQUENCE LISTING

<110> WORMSER, URI
<120> POLYPEPTIDES DERIVED FROM -1 ANTITRYPSIN AND METHODS OF USE THEREOF
<130> WORM-7253-PC
<150> US 61/378,414
   <151> 2010-08-31
<150> US 61/425,038
   <151> 2010-12-20
<160> 4
<170> PatentIn version 3.5
<210> 1
   <211> 37
   <212> PRT
   <213> Mus musculus
<400> 1
   Tyr Ser Met Pro Pro Ile Val Arg Phe Asp His Pro Phe Leu Phe Ile
   1               5                   10                  15
   Ile Phe Glu Glu His Thr Gln Ser Pro Leu Phe Val Gly Lys Val Val
               20                  25                  30
   Asp Pro Thr His Lys
           35
<210> 2
   <211> 36
   <212> PRT
   <213> Mus musculus
<400> 2
   Ser Met Pro Pro Ile Val Arg Phe Asp His Pro Phe Leu Phe Ile Ile
   1               5                   10                  15
   Phe Glu Glu His Thr Gln Ser Pro Leu Phe Val Gly Lys Val Val Asp
               20                  25                  30
   Pro Thr His Lys
           35
<210> 3
   <211> 35
   <212> PRT
   <213> Mus musculus
<400> 3
   Met Pro Pro Ile Val Arg Phe Asp His Pro Phe Leu Phe Ile Ile Phe
   1               5                   10                  15
   Glu Glu His Thr Gln Ser Pro Leu Phe Val Gly Lys Val Val Asp Pro
               20                  25                  30
   Thr His Lys
           35
<210> 4
   <211> 35
   <212> PRT
   <213> Mus musculus
<400> 4
   Ser Met Pro Pro Ile Val Arg Phe Asp His Pro Phe Leu Phe Ile Ile
   1               5                   10                  15
   Phe Glu Glu His Thr Gln Ser Pro Leu Phe Val Gly Lys Val Val Asp
               20                  25                  30
   Pro Thr His
           35